# EUROPEAN PATENT SPECIFICATION

(11) **EP 2 421 520 B1**
(45) Date of publication and mention of the grant of the patent: **16.01.2013**
(21) Application number: 10717816.2
(22) Date of filing: 19.04.2010
(51) Int. Cl.: A61P 11/02, A61K 31/00, A61K 31/047, A61K 31/19, A61K 31/70, A61K 31/715, A61K 38/38, A61K 47/26, A61K 47/48

(54) **ALBUMIN AND/OR MANNITOL FOR THE TREATMENT OF NASAL POLYPOSIS**
ALBUMIN UND/ODER MANNITOL ZUR BEHANDLUNG VON NASENPOLYPEN
ALBUMINE ET/OU MANNITOL POUR LE TRAITEMENT DE LA POLYPOSE NASALE

(30) Priority: 20.04.2009 IT RM20090180
(43) Date of publication of application: 29.02.2012
(73) Proprietor: D.M.G. Italia Srl, 00040 Pomezia (RM) (IT)
(72) Inventor: MERCURI, Luigi, I-00040 Pomezia RM (IT)
(74) Representative: Sarpi, Maurizio
(86) International application number: PCT/IB2010/051703
(87) International publication number: WO 2010/122482

(56) References cited:
- WO-A-2006/097456
- WO-A-2009/063367
- US-A1- 2004 258 757
- US-A1- 2009 017 090
- DATABASE WPI Week 199729 Thomson Scientific, London, GB; AN 1997-311179 XP002552879 & CN 1 104 533 A (LIU T) 5 July 1995 (1995-07-05)

## Description

The present invention relates to osmotic agents for use in treating nasal polyposis and to pharmaceutical compositions for the same use.

### STATE OF PRIOR ART

The nasal polyposis is a chronical disease of the nasal mucosa which is noticed in a percentage comprised between 1 and 4% of the population and about two thirds of these subjects are men. Such incidence increases considerably upon taking into account some groups of people with different nasal pathologies. The nasal polyposis can appear at any age and it can be connected to pathologies such as allergies, the chronic sinusitis, the intolerance towards analgesics or the mucoviscidosis and it appears with the formation of extroflexions at the level of the nasal mucosa.

Nasal polyps, extroflexions at the level of the nasal mucosa, usual with a round shape or a pear-like shape, forming initially in one of the paranasal sinuses in order then to develop even in the nasal cavity. They are pockets constituted by tissue with high liquid content and infiltrating cells (that is cells of the immune system), thereamong mastocytes, eosinophilic granulocytes, lymphocytes and plasmacells, which appear coated by ciliated epithelium of the aerial routes wherein it comes up. The most frequent seat wherein nasal polyps come up is the mucosa situated along the side wall of the nose. Another, less common seat is the rear portion of the nose, wherein the polyps connect to the epipharynx.

The inflamed mucosa transforms into a pale excess tissue, with a gelatinous and translucent aspect.

In past, the nasal polyps were surgically removed without resorting to any medical treatment. The surgical treatment has both the disadvantage of being an invasive technique and of being not always resolving. In fact, even after the surgical removal often pharmacological therapies are provided, aimed at avoiding a new onset of the disease. Currently, the tendency is to prepare conservative therapeutic strategies in order to improve as much as possible the life quality of the patient. By following such methodological setting, the therapy of the nasal polyposis first of all provides a pharmacological treatment, whereas the surgical intervention is performed only when the patients do not react to drugs.

The most widespread pharmacological therapies are based upon corticosteroids, such as beclomethasone dipropionate or flunisolide, generally administered by aerosol. The disadvantages of therapies based upon these compounds are the onset of reactions of local hypersensibility and the impossibility of being used in a prolonged way. Recently, treatments based upon non steroidal anti-inflammatory drugs have also been proposed (patent WO 9703659-A). Treatments against the nasal polyposis based upon this type of compounds are still in experimental phase and the real efficiency thereof upon patients suffering from said pathology has not yet been demonstrated.

In the light of the disadvantages mentioned above, the need for proposing new treatments for this disease, not having the disadvantages of the corticosteroid drugs, was felt very much.

The invention object of the present invention is based upon the surprising finding that the use of at least one osmotic agent in the seat wherein the nasal polyposis appears determines a great reduction or elimination of the symptoms and troubles associated to said pathology.

The present invention relates then to one or more osmotic agents for use in treating nasal polyposis and to pharmaceutical compositions containing thereof. By way of example, but not as a limiting example, the osmotic agent quantity comprised in said pharmaceutical compositions is between 30 and 50% by weight. The pharmaceutical compositions comprising one or more osmotic agents for use in treating the nasal polyposis and substances with anti-inflammatory activity are the additional object of the invention. The compositions contain usual additives and/or excipients and they can be implemented under the form of ointment, spray, powder, solution, gel, emulsion, cream.

The present invention in treating the nasal polyposis has the following advantages with respect to the known art:
It limitates or eliminates the use of pharmacological substances with unwished secondary effects such as for example corticosteroid anti-inflammatory drugs;
it provides an alternative or however it delays the resort to the surgical operation which is an invasive treatment;
it provides a treatment without side effects.

### DETAILED DESCRIPTION OF THE INVENTION

The present invention relates to one or more osmotic agents according to the claims for use in treating nasal polyposis.

Within the scope of the present invention under the term osmotic agent one or more compounds, are defined according to the claims. Their use in treating patients affected by nasal polyposis causes the formation of an osmotic gradient sufficient to reduce the inner pressure of the polyps themselves.

Furthermore, the oncotic agents are equivalent to the osmotic agents defined previously.

The invention could be used in treating the nasal polyposis as replacement or association to other pharmacological and/or surgical treatments. As already described previously a very widespread treatment against the nasal polyposis is the surgical removal of the polyps themselves. The invention could be used both as alternative treatment to a surgical treatment and as treatment preceding a possible surgical treatment and after the surgical treatment itself.

Some osmotic agents for treating the nasal polyposis according to the invention are designated hereinafter: mannitol, and/or albumin, optionally in combination with glycerol, malto-dextrin and sorbitol. The person skilled in the art based upon the examples and the definition given in this case is able to designate additional osmotic agents. According to the present invention the preferred osmotic agent used for treating the nasal polyposis is albumin, a protein of the blood plasma produced by the liver with molecular weight of about 69000 Dalton. Albumin is essential for regulating and maintaining the oncotic pressure, that is the osmotic pressure necessary for the correct distribution of the body liquids in the intravascular compartments and in the tissues. In case of a low oncotic pressure there is the formation of an oedema, a liquid excess in the tissues.

The use of a macromolecule such as albumin aims at creating a draining force for attracting liquid from the polyp tissue and therefore, in the end, for reducing the mass of the same. The mechanism is then the one opposite to what takes place at physiological level.

Mannitol, often called mannite, is an achiral polyalcohol, with seven hydroxylic groups at level of the aliphatic chain constituted by seven saturated atoms of carbon. The mannitol is used broadly in the pharmaceutical field since it has diuretic properties. In particular, it belongs to the class of the osmotic diuretics.

Therefore the objects of the present invention are compositions comprising albumin as osmotic agent and compositions comprising mannitol as osmotic agent for use. In a particularly advantageous embodiment of the invention an osmotic agent is utilized for use in treating nasal polyposis constituted by mannitol and albumin.

In a preferred embodiment of the compositions according to the present invention the osmotic agent constituted by mannitol and albumin is present in a percentage by weight comprised between 30 and 50%. Additional objects of the invention are pharmaceutical compositions comprising osmotic agents for use as described previously and one or more compounds with anti-inflammatory activity. Advantageously, the compositions of the present description can comprise one ore more anti-inflammatory drugs such as for example the glycyrrhizic acid or the glycyrrhetinic acid. The glycyrrhizic acid is a natural triterpenic acid and it represents the active principle of the liquorice extract, apart from the anti-inflammatory properties it has emollient, decongesting and bacteriostatic properties. The glycyrrhetinic acid is obtained by hydrolysis of the glycyrrhizic acid and like this one, apart from the anti-inflammatory properties, it has emollient, decongesting and bacteriostatic properties. Typical quantities of glycyrrhizic acid or glycyrrhetinic acid, expressed as percentage by weight, which can be used in the composition of the invention are 1-5%. In the compositions of the invention both isomers of the glycyrrhizic acid and of the glycyrrhetinic acid can be used, that is the 18-beta-glycyrrhizic acid, the 18-alpha-glycyrrhizinic acid, the 18-beta-glycyrrhetinic acid, the 18-alpha-glycyrrhetinic acid and the salts thereof..

The pharmacologically acceptable excipients to be used in the composition of the invention could be determined easily by the person skilled in the art by following standards known in the field of the pharmaceutical prior art. Examples of pharmaceutically acceptable excipients are caulino, maltodextrose, magnesium stearate, ascorbic acid, vaseline oil. The compositions could further include one or more aromatizing agents, emulsifying agents, dispersion agents, stabilizing agents, solubilizing agents or preservatives which the person skilled in the art, by following standard protocols known in the field of the pharmaceutical prior art, could be able to choose in a suitable way.

The compositions of the invention can be implemented in one of the following forms: creams, liquid solutions, gels, ointments, sprays, suspensions, emulsions, powders, granulates. The choice of the form and of the implementation of said compositions could be easily determined by the person skilled in the art by following standard protocols known in the field of the pharmaceutical prior art.

The compositions could be produced by following standard protocols known in the field of the pharmaceutical art.

By way of example the processes for implementing the invention in the form of ointment, gel, spray and powder are shown hereinafter.

To implement the invention in the ointment formulation the process shown hereinafter was used. Heating white vaseline up to a temperature of about 45°-50°C, add the vaseline oil, BHT, the chlorobutanol (previously micronized to ease the solubization thereof) and turn off the heating. At this point one ore more osmotic agents in powder, such as for example albumin and/or mannitol, are added by keeping the product under constant stirring. When the product temperature has decreased up to around 30-35°C vitamin E, in form of acetate salt, and peppermint oil are added. The result of this preparation is a consistent ointment with white ivory colour tending to yellow. In an additional embodiment of the invention in the ointment formulation the same process described previously is used, but after adding one ore more osmotic agents even one ore more anti-inflammatory agents, such as for example the glycyrrhetinic acid or one of the salts thereof, is added.

To implement the invention in the spray formulation the process shown hereinafter is used. Polisorbate-20 and the aromatizing agent are weighed, then they are mixed together and the purified water and then one or more osmotic agents, such as for example albumin and/or mannitol, are added. Then glycerin is added and one waits for until complete solubilization. At this point one or more anti-inflammatory agents, such as glycyrrhizined potassium, are added and one waits for the complete solubilization thereof. The solution is neutralized by means of adding sodium hydroxide, the preservatives (EDTA and N-hydroxymethylglycinate) are added and at last the buffer salts are added. In this way a limpid solution is obtained, with a colour tending to yellow with pH 7.0 and with an osmolarity value greater than or equal to 1000 mOsM/L.

In order to implement the invention in the gel formulation the process shown hereinafter is used. Polisorbate-20 and the flavouring agent are weighed and then they are mixed together and the purified water is added and then one or more osmotic agents are added, such as for example albumin and/or mannitol. Then, glycerin is added (in order to increase the wetting capability of the two powders) and one waits for until complete solubilization. At this point one or more anti-inflammatory agents, such as glycyrrhizined potassium, are added and one waits for the complete solubilization thereof. Then Carbopol is added, after having dispersed the latter the preservatives, for example EDTA and N-hydroxymethylglycinate, are added and, at last, it is brought to pH 7 with sodium hydroxide drop by drop. In this way a viscous gel with a colour tending to yellow with pH 7.0 and with an osmolarity value greater or equal to 1000 mOsM/L is obtained.

In order to implement the invention in the powder formulation the process shown hereinafter is used. The several formulation components which are shown in the examples 19-26, are properly mixed with a mixer of Blender kind. The crystals of mannitol, lactose, glycyrrhized potassium and malto-dextrins are properly micronized in order to obtain a size comprised between 10-100 micron. The malto-dextrins are used both as osmotic agents and to improve sliding of powders. Subsequently, the four micronized components are mixed with the proper quantity of albumin, the different quantities of albumin which can be used are shown in the examples 19-26. In order to further improve the dispersibility of the composition, all formulation components are solubilized and/or dispersed in water and subsequently lyophilized. A perfect mixture is then obtained. The lyophilized powder which is obtained can be used also for a spray dispenser.

Each element specifically designated in the present application is meant as exemplifying and not limiting element, therefore it cannot be excluded from the conferred protection scope without altering the essence of the invention.

The invention will be illustrated hereinafter by means of examples.

### EXAMPLES

The following examples are only illustrative. The compositions shown hereinafter are produced by means of the processes described previously. The quantities of the single compounds are expressed as percentages by weight.

### Example 1

In a particular embodiment the invention has the following ointment formulation:

| Ingredients | % p/w |
|---|---|
| Vaseline oil | 28.5 |
| White Vaseline | 25.94 |
| Albumin | 40 |
| Chlorobutanol | 0.5 |
| Vit E acetate | 0.5 |
| BHT | 0.05 |
| Peppermint oil | 0.01 |

### Example 2

In a particular embodiment the invention has the following ointment formulation:

| Ingredients | % p/w |
|---|---|
| Vaseline oil | 28.5 |
| White Vaseline | 25.94 |
| Mannitol | 40 |
| Chlorobutanol | 0.5 |
| VitE acetate | 0.5 |
| BHT | 0.05 |
| Peppermint oil | 0.01 |

### Example 3

In a particular embodiment the invention has the following ointment formulation:

| Ingredients | % p/w |
|---|---|
| Vaseline oil | 28.5 |
| White Vaseline | 25.94 |
| Albumin | 10 |
| Mannitol | 30 |
| Chlorobutanol | 0.5 |
| VitE acetate | 0.5 |
| BHT | 0.05 |
| Peppermint oil | 0.01 |

### Example 4

In a particular embodiment the invention has the following ointment formulation

| Ingredients | % p/w |
|---|---|
| Vaseline oil | 28.5 |
| White Vaseline | 25.94 |
| Albumin | 30 |
| Mannitol | 10 |
| Chlorobutanol | 0.5 |
| VitE acetate | 0.5 |
| BHT | 0.05 |
| Peppermint oil | 0.01 |

### Example 5

In a particular embodiment the invention has the following ointment formulation:

| Ingredients | % p/w |
|---|---|
| Vaseline oil | 28 |
| White Vaseline | 25.94 |
| Albumin | 10 |
| Mannitol | 30 |
| Dipotassium glycyrrhizinate | 5 |
| Chlorobutanol | 0.5 |
| VitE acetate | 0.5 |
| BHT | 0.05 |
| Peppermint oil | 0.01 |

### Example 6

In a particular embodiment the invention has the following ointment formulation:

| Ingredients | % p/w |
|---|---|
| Vaseline oil | 28 |
| White Vaseline | 25.94 |
| Albumin | 30 |
| Mannitol | 10 |
| Dipotassium glycyrrhizinate | 5 |
| Chlorobutanol | 0.5 |
| VitE acetate | 0.5 |
| BHT | 0.05 |
| Peppermint oil | 0.01 |

### Example 7

In a particular embodiment the invention has the following ointment formulation:

| Ingredients | % p/w |
|---|---|
| Vaseline oil | 28 |
| White Vaseline | 25.94 |
| Mannitol | 40 |
| Dipotassium glycyrrhizinate | 5 |
| Chlorobutanol | 0.5 |
| VitE acetate | 0.5 |
| BHT | 0.05 |
| Peppermint oil | 0.01 |

### Example 8

In a particular embodiment the invention has the following ointment formulation:

| Ingredients | % p/w |
|---|---|
| Vaseline oil | 28 |
| White Vaseline | 25.94 |
| Albumin | 40 |
| Dipotassium glycyrrhizinate | 5 |
| Chlorobutanol | 0.5 |
| Vit E acetate | 0.5 |
| BHT | 0.05 |
| Peppermint oil | 0.01 |

### Example 9

In a particular embodiment the Invention has the following spray formulation:

| Ingredients | % p/w |
|---|---|
| Albumin | 10 |
| Mannitol | 30 |
| Glycerin | 2 |
| Sodium phosphate monobasic | 0.15 |
| Sodium phosphate dibasic | 0.40 |
| EDTA disodium dihydrate | 0.1 |
| Polysorbate 20 | 0.2 |
| Strong mint aroma | 0.01 |
| N-hydroxymethylglycinate | 0.05 |
| Sodium hydroxide | 0.155 |
| Demineralized-deionized water | as much as suffices at 100g |

### Example 10

In a particular embodiment the invention has the following spray formulation:

| Ingredients | % p/p |
|---|---|
| Albumin | 10 |
| mannitol | 30 |
| Dipotassium glycyrrhizinate | 5 |
| Glycerin | 2 |
| Sodium phosphate monobasic | 0.15 |
| Sodium phosphate dibasic | 0.40 |
| EDTA disodium dihydrate | 0.1 |
| Polysorbate 20 | 0.2 |
| Strong mint aroma | 0.01 |
| N-hydroxymethylglycinate | 0.05 |
| Sodium hydroxide | 0.155 |
| Demineralized-deionized water | as much as suffices at 100g |

### Example 11

In a particular embodiment the invention has the following spray formulation:

| Ingredients | % p/w |
|---|---|
| Albumin | 30 |
| Mannitol | 10 |
| Glycerin | 2 |
| Sodium phosphate monobasic | 0.15 |
| Sodium phosphate dibasic | 0.40 |
| EDTA disodium dihydrate | 0.1 |
| Polysorbate 20 | 0.2 |
| Strong mint aroma | 0.01 |
| N-hydroxymethylgiycinate | 0.05 |
| Sodium hydroxide | 0.155 |
| Demineralized-deionized water | as much as suffices at 100g |

### Example 12

In a particular embodiment the invention has the following spray formulation:

| Ingredients | % p/w |
|---|---|
| Albumin | 30 |
| Mannitol | 10 |
| Dipotassium glycyrrhizinate | 5 |
| Glycerin | 2 |
| Sodium phosphate monobasic | 0.15 |
| Sodium phosphate dibasic | 0.40 |
| EDTA disodium dihydrate | 0.1 |

| | |
|---|---|
| Polysorbate 20 | 0.2 |
| Strong mint aroma | 0.01 |
| N-hydroxymethylglycinate | 0.05 |
| Sodium hydroxide | 0.155 |
| Demineralized-deionized water | as much as suffices at 100g |

### Example 13

In a particular embodiment the invention has the following spray formulation:

| Ingredients | % p/w |
|---|---|
| Albumin | 20 |
| Mannitol | 20 |
| Dipotassium glycyrrhizinate | 5 |
| Glycerin | 2 |
| Sodium phosphate monobasic | 0.15 |
| Sodium phosphate dibasic | 0.40 |
| EDTA disodium dihydrate | 0.1 |
| Polysorbate 20 | 0.2 |
| Strong mint aroma | 0.01 |
| N-hydroxymethylglycinate | 0.05 |
| Sodium hydroxide | 0.155 |
| Demineralized-deionized water | as much as suffices at 100g |

### Example 14

In a particular embodiment the invention has the following spray formulation:

| Ingredients | % p/w |
|---|---|
| Albumin | 30 |
| Mannitol | 10 |
| Dipotassium glycyrrhizinate | 5 |
| glycerin | 2 |
| Sodium phosphate monobasic | 0.15 |
| Sodium phosphate dibasic | 0.40 |
| EDTA disodlum dihydrate | 0.1 |
| Polysorbate 20 | 0.2 |
| Strong mint aroma | 0.01 |
| N-hydroxymethylglycinate | 0.05 |
| Sodium hydroxide | 0.155 |
| Demineralized-deionized water | as much as suffices at 100g |

### Example 15

In a particular embodiment the invention has the following spray formulation:

| Ingredients | % p/w |
|---|---|
| Albumin | 40 |
| Dipotassium glycyrrhizinate | 5 |
| glycerin | 2 |
| Sodium phosphate monobasic | 0.15 |
| Sodium phosphate dibasic | 0.40 |
| EDTA disodium dihydrate | 0.1 |
| Polysorbate 20 | 0.2 |
| Strong mint aroma | 0.01 |
| N-hydroxymethylglycinate | 0.05 |
| Sodium hydroxide | 0.155 |
| Demineralized-deionized water | as much as suffices at 100g |

### Example 16

In a particular embodiment the invention has the following spray formulation:

| Ingredients | % p/w |
|---|---|
| Mannitol | 40 |
| Dipotassium glycyrrhizinate | 5 |
| glycerin | 2 |
| Sodium phosphate monobasic | 0.15 |
| Sodium phosphate dibasic | 0.40 |
| EDTA disodium dihydrate | 0.1 |
| Polysorbate 20 | 0.2 |
| Strong mint aroma | 0.01 |
| N-hydroxymethylglycinate | 0.05 |
| Sodium hydroxide | 0.155 |
| Demineralized-deionized water | as much as suffices at 100g |

### Example 17

In a particular embodiment the invention has the following gel formulation.

| Ingredients | % p/w |
|---|---|
| Albumin | 10 |
| Mannitol | 30 |
| Dipotassium glycyrrhizinate | 5 |
| Glycerin | 2 |
| Carbopol | 0.15 |
| EDTA disodium dihydrate | 0.1 |
| Polysorbate 20 | 0.2 |
| Strong mint aroma | 0.01 |
| N-hydroxymethylglycinate | 0.002 |
| Sodium hydroxide | 0.155 |
| Demineralized-deionized water | as much as suffices at 100g |

### Example 18

In a particular embodiment the invention has the following gel formulation:

| Ingredients | % p/w |
|---|---|
| Albumin | 20 |
| Mannitol | 20 |
| Dipotassium glycyrrhizinate | 5 |
| Glycerin | 2 |
| Carbopol | 0.15 |
| EDTA disodium dihydrate | 0.1 |
| Polysorbate 20 | 0.2 |
| Strong mint aroma | 0.01 |
| N-hydroxymethylglycinate | 0.002 |
| Sodium hydroxide | 0.155 |
| Demineralized-deionized water | as much as suffices at 100g |

### Example 19

In a particular embodiment the invention has the following gel formulation:

| Ingredients | % p/w |
|---|---|
| Albumin | 30 |
| Mannitol | 10 |
| Dipotassium glycyrrhizinate | 5 |
| Glycerin | 2 |
| Carbopol | 0.15 |
| EDTA disodium dihydrate | 0.1 |
| Polysorbate 20 | 0.2 |
| Strong mint aroma | 0.01 |
| N-hydroxymethylglycinate | 0.002 |
| Sodium hydroxide | 0.155 |
| Demineralized-deionized water | as much as suffices at 100g |

### Example 20

In a particular embodiment the invention has the following gel formulation:

| Ingredients | % p/w |
|---|---|
| Albumin | 40 |
| Dipotassium glycyrrhizinate | 5 |
| Glycerin | 2 |
| Carbopol | 0.15 |
| Sodium phosphate monobasic | 0.15 |
| Sodium phosphate dibasic | 0.40 |
| EDTA disodium dihydrate | 0.1 |
| Polysorbate 20 | 0.2 |
| Strong mint aroma | 0.01 |
| N-hydroxymethylglycinate | 0.002 |
| Sodium hydroxide | 0.155 |
| Demineralized-deionized water | as much as suffices at 100g |

### Example 21

In a particular embodiment the invention has the following gel formulation:

| Ingredients | % p/w |
|---|---|
| Mannitol | 40 |
| Dipotassium glycyrrhizinate | 5 |
| Glycerin | 2 |
| Carbopol | 0.15 |
| EDTA disodium dihydrate | 0.1 |
| Polysorbate 20 | 0.2 |
| Strong mint aroma | 0.01 |
| N-hydroxymethylglycinate | 0.002 |
| Sodium hydroxide | 0.155 |
| Demineralized-de ionized water | as much as suffices at 100g |

### Example 22

In a particular embodiment the invention has the following powder formulation:

| Ingredients | % p/w |
|---|---|
| Albumin | 90 |
| Mannitol | 30 |
| Dipotassium glycyrrhizinate | 5 |
| Lactose | 50 |
| Maltodextrins | 5 |

### Example 23

In a particular embodiment the invention has the following powder formulation:

| Ingredients | % p/w |
|---|---|
| Albumin | 30 |
| Mannitol | 10 |
| Dipotassium glycyrrhizinate | 5 |
| Lactose | 50 |
| Maltodextrins | 5 |

### Example 24

In a particular embodiment the invention has the following powder formulation:

| Ingredients | % p/w |
|---|---|
| Mannitol | 40 |
| Dipotassium glycyrrhizinate | 5 |
| Lactose | 50 |
| Maltodextrins | 5 |

### Example 25

In a particular embodiment the invention has the following powder formulation:

| Ingredients | % p/w |
|---|---|
| Albumin | 40 |
| Dipotassium glycyrrhizinate | 5 |
| Lactose | 50 |
| Maltodextrins | 5 |

### Example 26

In a particular embodiment the invention has the following powder formulation:

| Ingredients | % p/w |
|---|---|
| Albumin | 10 |
| Dipotassium glycyrrhizinate | 5 |
| Mannitol | 30 |
| Lactose | 50 |

### Example 27

In a particular embodiment the invention has the following powder formulation:

| Ingredients | % p/w |
|---|---|
| Albumin | 30 |
| Dipotassium glycyrrhizinate | 5 |
| Mannitol | 10 |
| Lactose | 50 |
| Maltodextrins | 5 |

### Example 28

In a particular embodiment the invention has the following powder formulation:

| Ingredients | % p/w |
|---|---|
| Mannitol | 40 |
| Dipotassium glycyrrhizinate | 5 |
| Lactose | 50 |
| Maltodextrins | 5 |

### Example 29

In a particular embodiment the invention has the following powder formulation:

| Ingredients | % p/w |
|---|---|
| Albumin | 40 |
| Dipotassium glycyrrhizinate | 5 |
| Lactose | 50 |
| Maltodextrins | 5 |

### Clinical study on patients affected by nasal polyposis

Thirteen patients (with age comprised between 16 and 66 years old, 7 women and 6 men) affected by nasal polyposis have been treated for six months, with topical application of an ointment comprising albumin and mannitol according to the formula of the example number 4. Nine of the enrolled subjects were affected by polyposes recidivous; that is already subjected to one ore more treatments of functional endoscopic surgery which have shown in different times disease relapse. Two subjects of female sex of the sample showed an allergy against the acetylsalicylic acid. None of the subjects was affected by cystic fibrosis. It is important not testing patients affected by cystic fibrosis since the cystic fibrosis is a disease causing inflammation of the glands in the general sense and therefore in order to have a more correct and clear picture in the evaluation of the composition efficiency, the patients with cystic fibrosis were excluded from the test. In all patients there was a symptomatological improvement, the improvement was documented as from the therapy beginning, after 3 months and after 6 months of treatment. Regarding the extent of the polyposis, recidivous or not, the patients were evaluated with nasal endoscopy and they were classified according to the disease stages shown in the article of ROLAND GIGER and al. Otolaryngology Head and Neck Surgery 2004.
The sample of patients showed the following distribution in the check after three and six months:

| | Nr. subj. (after 3 months) | Nr. subj. (after 6 months) |
|---|---|---|
| I STAGE | 3 | 6 |
| II STAGE | 5 | 5 |
| | | |
| III STAGE | 4 | 2 |
| IV STAGE | 1 | 0 |

The distribution after 6 months shows a fewer number of individuals in the more advanced stages of the disease. During the treatment period no side effects were found.

### BIBLIOGRAPHY

WO 9703659-A. Use of an H+, K+-atpase inhibitor in the treatment of nasal polyps. Chronic panrhinosinusitis without nasal polyps: Long-term outcome after functional endoscopic sinus surgery ROLAND GIGER, PAVEL DULGUEROV, DIDIER QUINODOZ, DANIEL LEUBA, BASILE NICOLAS LANDIS, JEAN-SILVAIN LACROIX, and JEAN PAUL FRIEDRICH Otolaryngology Head and Neck Surgery Volume 131 Number 4 2004.

## Claims

1. A pharmaceutical composition for topic use in treating nasal polyps comprising as active agent albumin and/or mannitol.

2. Composition for use according to claim 1 **characterized in that** it further comprises other osmotic agents such as maltodextrin, glycerol and sorbitol.

3. Composition for use according to claim 1 **characterized in that** the active agent constituted by mannitol and/or albumin is present in a percentage by weight between 30 and 50%.

4. Composition for use according to claim 1-3 **characterized in that** it further comprises one or more compounds with anti-inflammatory activity, preferably the glycyrrhizic acid or the glycyrrhetinic acid, their isomers the 18-beta-glycyrrhizic acid, the 18-alpha-glycyrrhizinic acid, the 18-beta-glycyrrhetinic acid, the 18-alpha-glycyrrhetinic acid, and the salts thereof.

5. Composition for use according to claim 4 **characterized in that** the compounds with anti-inflammatory activity are present in a percentage by weight between 1 and 5%.

6. Composition for use according to any preceding claim **characterized in that** it further comprises acceptable excipients, aromatizing agents, emulsifying agents, dispersion agents, stabilizing agents, solubilizing agents, preservatives.

7. The pharmaceutical composition for topic use in treating nasal polyps according to anyone of preceding claims in the form of suspension, emulsion, cream, spray, ointment, powder gel or liquid solution.

## Patentansprüche

1. Pharmazeutische Zusammensetzung für die topische Verwendung in der Behandlung von nasalen Polypen umfassend als Wirkstoff Albumin und/oder Mannitol.

2. Zusammensetzung zur Verwendung gemäß Anspruch 1, **dadurch gekennzeichnet, dass** sie weiter andere osmotische Wirkstoffe wie Maltodextrin, Glycerol und Sorbitol umfasst.

3. Zusammensetzung zur Verwendung gemäß Anspruch 1, **dadurch gekennzeichnet, dass** der Wirkstoff bestimmt durch Mannitol und/oder Albumin in einem Gewichtsanteil zwischen 30 % und 50 % enthalten ist.

4. Zusammensetzung zur Verwendung gemäß Anspruch 1 bis 3, **dadurch gekennzeichnet, dass** sie weiter eine oder mehrere Verbindungen mit entzündungshemmender Aktivität, bevorzugt Glycyrrhizinsäure oder Glycyrrhetinsäure, ihre Isomere 18-beta-Glycyrrhizinsäure, 18-alpha-Glycyrrhizinsäure, 18-beta-Glycyrrhetinsäure, 18-alpha-Glycyrrhetinsäure und Salze davon umfasst.

5. Zusammensetzung zur Verwendung gemäß Anspruch 4, **dadurch gekennzeichnet, dass** die Verbindungen mit entzündungshemmender Aktivität in einem Gewichtsanteil zwischen % und 5 % enthalten sind.

6. Zusammensetzung zur Verwendung gemäß einem der vorstehenden Ansprüche, **dadurch gekennzeichnet, dass** sie weiter verträgliche Hilfsstoffe, aromatisierende Stoffe, Emulgatoren, Dispersionsmittel, Stabilisierungsmittel, Lösungshilfsmittel, Konservierungsmittel umfasst.

7. Pharmazeutische Zusammensetzung für die topische Verwendung in der Behandlung von nasalen Polypen gemäß einem der vorstehenden Ansprüche in der Form einer Suspension, Emulsion, Creme, Spray, Salbe, Pulvergel oder flüssigen Lösung.

## Revendications

1. Composition pharmaceutique pour l'usage topique dans le traitement de polypes nasaux comprenant comme agent actif l'albumine et/ou le mannitol.

2. Composition pour l'usage selon la revendication 1, **caractérisée en ce qu'**elle comprend en outre d'autres agents osmotiques tels que la maltodextrine, le glycérol et le sorbitol.

3. Composition pour l'usage selon la revendication 1, **caractérisée en ce que** l'agent actif constitué par le mannitol et/ou l'albumine est présent dans un pourcentage en poids compris entre 30 et 50%.

4. Composition pour l'usage selon la revendication 1-3, **caractérisée en ce qu'**elle comprend en outre un ou plusieurs composés avec activité anti-inflammatoire, de préférence l'acide glycyrrhizique ou l'acide glycyrrhétinique, leurs isomères l'acide 18-bêta-glycyrrhizique, l'acide 18-alpha-glycyrrhizinique, l'acide 18-bêta-glycyrrhétinique, l'acide 18-alpha-glycyrrhétinique et leurs sels.

5. Composition pour l'usage selon la revendication 4, **caractérisée en ce que** les composés activité anti-inflammatoire sont présents dans un pourcentage en poids compris entre 1 et 5%.

6. Composition pour l'usage selon une quelconque revendication précédente, **caractérisée en ce qu'**elle comprend en outre des excipients acceptables, agents aromatiques, agents émulsifiants, agents de dispersion, agents de stabilisation, agents de dissolution, agents conservateurs.

7. Composition pharmaceutique pour l'usage topique dans le traitement de polypes nasaux selon l'une quelconque des revendications précédentes sous la forme de suspension, émulsion, crème, aérosol, pommade, poudre, gel ou solution liquide.
